# EUROPEAN PATENT SPECIFICATION

(11) **EP 1 115 879 B1**
(45) Date of publication and mention of the grant of the patent: **18.06.2008**
(21) Application number: 99950543.1
(22) Date of filing: 23.09.1999
(51) Int. Cl.: C12N 15/86, C12N 15/12, C12N 15/48, C12N 5/10, C12N 7/01, A61K 48/00

(54) **RETROVIRAL PARTICLES PROTECTED AGAINST COMPLEMENT MEDIATED DESTRUCTION**
RETROVIRALE PARTIKEL, GESCHÜTZT GEGEN KOMPLEMENTVERMITTELTE LYSE
PARTICULES RETROVIRALES PROTEGEES CONTRE LA DESTRUCTION DONT LA MEDIATION EST ASSUREE PAR LE COMPLEMENT

(30) Priority: 23.09.1998 DK 119598
(43) Date of publication of application: 18.07.2001
(73) Proprietor: Institut für Virologie Teilrechtsfähiges Institut an der Veterinärmedizinischen Universität Wien, 1210 Wien (AT)
(72) Inventor: GÜNZBURG, Walter, A-2340 Mödling (AT); SALMONS, Brian, A-2340 Mödling (AT); BAUMANN, Jörg, A-1190 Wien (AT)
(74) Representative: Schwarz, Albin
(86) International application number: PCT/EP1999/007083
(87) International publication number: WO 2000/017374

(56) References cited:
- WO-A-99/27121
- TAKEUCHI Y ET AL: "TYPE C RETROVIRUS INACTIVATION BY HUMAN COMPLEMENT IS DETERMINED BYBOTH THE VIRAL GENOME AND THE PRODUCER CELL" JOURNAL OF VIROLOGY,US,THE AMERICAN SOCIETY FOR MICROBIOLOGY, vol. 68, no. 12, December 1994 (1994-12), page 8001-8007 XP000569528 ISSN: 0022-538X
- M SAIFUDDIN ET AL: "Human imunodeficiency virus type 1 incorporates both glycosyl phosphatidylinositol-anchored CD55 and CD59 and integral membrane CD46 at levels that protect from complement-mediated destruction" JOURNAL OF VIROLOGY,US,THE AMERICAN SOCIETY FOR MICROBIOLOGY, vol. 78, no. 78, 1997, page 1907-1911-1911 XP002100207 ISSN: 0022-538X
- SAIFUDDIN M (REPRINT) ET AL: "ROLE OF VIRION-ASSOCIATED GLYCOSYLPHOSPHATIDYLINOSITOL-LINKED PROTEINS CD55 AND CD59 IN COMPLEMENT RESISTANCE OF CELL LINE-DERIVED AND PRIMARY ISOLATES OF HIV-L" JOURNAL OF EXPERIMENTAL MEDICINE,JP,TOKYO, vol. 182, no. 2, 1 August 1995 (1995-08-01), page 501-509 XP002100208 ISSN: 0022-1007
- MARSCHANG P ET AL: "Decay-accelerating factor ( CD55 ) protects human immunodeficiency virus type 1 from inactivation by human complement" EUROPEAN JOURNAL OF IMMUNOLOGY,DE,WEINHEIM, vol. 25, no. 1, 1995, page 285-290 XP002100209 ISSN: 0014-2980
- G T SPEAR ET AL: "Host cell-derived complement control proteins CD55 and CD59 are incorporated into the virions of two unrelated enveloped viruses" JOURNAL OF IMMUNOLOGY,US,THE WILLIAMS AND WILKINS CO. BALTIMORE, vol. 155, 1995, page 4376-4381-4381 XP002100210 ISSN: 0022-1767
- D C MONTEFIORI ET AL: "Complement control proteins, CD46, CD55, and CD59, as common surface constituents of human and simian immunodeficiency viruses and possible targets for vaccine protection" VIROLOGY,US,ACADEMIC PRESS,ORLANDO, vol. 205, 1994, page 82-92-92 XP002100211 ISSN: 0042-6822
- BREUN S ET AL., : "Protection of MLV vector particles from human complement." BIOCHEMICAL AND BIOPHYSICAL RESEARCH COMMUNICATIONS, vol. 264 (1), 14 October 1999 (1999-10-14), page 1-5 XP000857844

## Description

The present invention relates to retroviral particles which are specifically applicable as safe gene transfer vehicles for targeted gene therapy and which are protected against complement mediated destruction in host organisms. The present invention also relates to a packaging cell producing said retroviral particles.

### Background of the invention

The use of retroviral vectors for gene therapy has received much attention and currently is the method of choice for the transferral of therapeutic genes in a variety of approved protocols both in the USA and in Europe (Kotani et al., 1994). However most of these protocols require that the infection of target cells with the retroviral vector carrying the therapeutic gene occurs *in vitro,* and successfully infected cells are then returned to the affected individual (Rosenberg et al., 1992; for a review see Anderson, 1992). Such *ex vivo* gene therapy protocols are ideal for correction of medical conditions in which the target cell population can be easily isolated (e.g. lymphocytes). Additionally the *ex vivo* infection of target cells allows the administration of large quantities of concentrated virus which can be rigorously safety tested before use.

Unfortunately, only a fraction of the possible applications for gene therapy involve target cells that can be easily isolated, cultured and then reintroduced. Additionally, the complex technology and associated high costs of *ex vivo* gene therapy effectively preclude its disseminated use world-wide. Future facile and cost-effective gene therapy will require an *in vivo* approach in which the viral vector, or cells producing the viral vector, are directly administered to the patient in the form of an injection or simple implantation of retroviral vector producing cells.

This kind of *in vivo* approach, of course, introduces a variety of new problems. First of all, and above all, safety considerations have to be addressed. Virus will be produced, possibly from an implantation of virus producing cells, and there will be no opportunity to precheck the produced virus. It is important to be aware of the finite risk involved in the use of such systems, as well as trying to produce new systems that minimize this risk.

For therapeutic purposes the retroviral vector itself is normally a modified vector in which the genes encoding for the viral proteins have been replaced by therapeutic genes and marker genes to be transferred to the target cell. Since the replacement of the genes encoding for the viral proteins effectively cripples the virus it must be rescued by a component which provides the missing viral proteins to the modified retrovirus. This component is regularly a cell line that produces large quantities of the viral proteins, however lacks the ability to produce replication competent virus. This cell line is known as the packaging cell line and consists of a cell line transfected with a second plasmid carrying the genes enabling the modified retroviral vector to be packaged.

To generate the packaged vector, the vector plasmid is transfected into the packaging cell line. Under these conditions the modified retroviral genome including the inserted therapeutic and marker genes is transcribed from the vector plasmid and packaged into the modified retroviral particles (recombinant viral particles). This recombinant virus is then used to infect target cells in which the vector genome and any carried marker or therapeutic genes becomes integrated into the target cell's DNA. A cell infected with such a recombinant viral particle cannot produce new viruses since no viral proteins are present in these cells. However, the DNA of the vector carrying the therapeutic and marker genes is integrated in the cell's DNA and can now be expressed in the infected cell.

It has been shown that retroviral vectors packaged in non-human cell derived envelope proteins are inactivated and lysed by human sera and that, thus, the effectivity of *in vivo* gene transfer using recombinant retroviruses and packaging cells transfected with a recombinant retroviral vector, respectively, is limited (Russell et al., 1995). It is well documented that during infection with most viruses, an immune response develops against the virus-encoded envelope protein(s) and that most viruses are inactivated and lysed by human complement (Welsh et al., 1975 and Cooper et al., 1976).

The complement system works via two pathways, the classic and the alternative. The classic complement response functions as a result of the formation of antigen-antibody complexes and is dependent on the presence of specific antibodies. Consequently, this type of reaction belongs to the acquired humoral immune response. The alternative pathway employs an antibody-independent foreign surface specific response. Both trigger a cascade, in which multiple proteins are involved, eventually leading to lysis of either the pathogen or the infected cell by activation of a membrane attack complex (MAC).

Recombinant virions produced by packaging cell lines that originate from non-primates are lysed by an antibody mediated complement response (Rother et al., 1996 and Takeuchi et al., 1996): The retroviral envelope, formed during budding, is composed of the retroviral envelope protein and the packaging cell membrane, respectively, and incorporates other surface proteins of said packaging cell. Non-primate cells express galactose α(1-3)-galactose terminal carbohydrate residues (α-gal) on their surface. This enzymatic modification cannot be carried out in primate cells since the gene encoding α(1-3)-galactosyltransferase has been inactived by mutation (Galili et al., 1988). However, the α-gal modification is found on human intestinal bacteria, and as a result more than 1% of circulating human B-lymphocytes produce antibodies that can recognize the α-gal epitope. Consequently, non-primate enveloped viruses that present this residue on their surface are rapidly bound by these pre-formed antibodies and then destroyed by the human complement system.

On the other hand, retroviruses originating from human cells, such as the human immunodeficiency virus 1 (HIV-1) and human T-cell leukaemia virus 1 (HTLV-1) are able to escape the complement system (Marschang et al., 1995 and Spear et al., 1995). When produced from a human cell these viruses will also contain human proteins, including human complement inhibitors. In humans, these inhibitors, which are found on haematopoetic, epithelial and endothelial cells, act as protection against the individual's own complement system. If, for example, such a cell has been infected by HIV-1, the complement inhibiting proteins that convey protection will also be present in the envelope of the virus after it buds from the cell (Saifuddin et al., 1997). The presence of such complement inhibitors in the viral envelope will protect it from the action of the complement system in subsequently infected humans.

Accordingly, human cell lines have been used for generating retroviral particles (Cosset et al., 1995). Nevertheless, considerable variations between the various cell lines screened for their ability to release retroviruses have been found and only few cell lines were able to produce retroviruses that were resistant to human serum. Additionally, it was found that viral particles generated with different envelopes showed significant differences in resistance and stability in human serum. Considering safety aspects, it is also not desired to use human cell lines for the generation of retroviruses which shall be used as vehicles for transfer of therapeutic genes into human target cells, since the genome of such cell lines may still comprise proviral genome which may evolve to replication-competent viruses. These viruses are, then, also released from the packaging cell line and transferred to the target cell. They may also modify other viruses, such as human immunodeficiency virus or endogenous viruses, by phenotypic and/ or genetic mixing in gene therapy recipients.

### Object of the invention

It is, thus, an object of the present invention to provide retroviral particles as safe gene transfer vehicles for targeted gene therapy which are protected against complement mediated destruction in host organisms.

### Detailed description of the invention

To achieve the foregoing and other objects, the present invention provides a murine packaging cell for producing retroviral particles, comprising a DNA construct coding for proteins required for a mouse leukemia virus (MLV) derived vector to be packaged and, additionally, a DNA construct coding for one or more complement human inhibitors, wherein one complement inhibitor is human CD59.

It was found by the inventors of the present invention that the complement inhibitor is indeed inserted into the membrane of the packaging cell. When generating viruses from such packaging cells it has been found that the complement inhibitor is inserted into the viral envelope, obviously due to the incorporation of the surface proteins of the packaging cell and, thus, of the complement inhibitor into the retroviral envelope during budding. Furthermore, it has been surprisingly found that these viruses are indeed protected against the complement system of the host organism. This result is in so far a surprising result as it was demonstrated by Takeuchi et al. (1994) that not only producer cell components but also viral components and the viral genome, respectively, determine the sensitivity of retroviruses to complement, specifically to the human complement.

According to the invention non-primate, specifically murine cells are used as packaging cells, and the DNA construct coding for the one or more complement inhibitors encodes the human, complement inhibitor CD59. Furthermore, the packaging cell may also comprise a DNA construct coding for homologous complement inhibitors. In this case, the amount of the complement inhibitor already incorporated into the packaging cell membrane is increased and, consequently, generated retroviral particles with enhanced protection against complement mediated destruction are obtained.

According to a preferred embodiment of the invention, the cell is PALSG/S.

Retroviral particles are produced by transfecting the packaging cell line according to the present invention with a retroviral vector comprising preferably one or more heterologous nucleic acid sequences, specifically coding for substances applicable in gene therapy, as e.g. marker genes, antiviral genes and/or antitumour genes. Considering safety aspects of *in vivo* application of such viruses, preferably safe gene transfer vehicles using the principle of promoter conversion typical for retroviruses are used (see PCT/EP95/03445):

The retroviral genome consists of an RNA molecule with the structure R-U5-gag-pol-env-U3-R. During the process of reverse transcription, the U5 region is duplicated at the right hand end of the generated DNA molecule, whilst the U3 region is duplicated and placed at the left hand end of the generated DNA molecule. The resulting structure U3-R-U5 is called LTR (Long Terminal Repeat) and is thus identical and repeated at both ends of the DNA structure or provirus. The U3 region at the left hand end of the provirus harbours the promoter. This promoter drives the synthesis of an RNA transcript initiating at the boundary between the left hand U3 and R regions and terminating at the boundary between the right hand R und U5 region. This RNA is packaged into retroviral particles and transported into the target cell to be infected. In the target cell the RNA genome is again reverse transcribed as described above.

In the promoter conversion vector the right hand U3 region is altered, but the normal left hand U3 structure is maintained; the vector can be normally transcribed into RNA utilizing the normal retroviral promoter located within the left hand U3 region. However, the generated RNA will only contain the altered right hand U3 structure. In the infected target cell, after reverse transcription, this altered U3 structure will be placed at both ends of the retroviral structure.

The altered region carries a polylinker instead of the U3 region. Thus, any promoter, including those directing tissue specific expression can be easily inserted. This promoter is then utilized exclusively in the target cell for expression of linked genes carried by the retroviral vector. Accordingly, in the packaging cell line the expression of the retroviral vector is regulated by the normal unselective retroviral promoter contained in the U3 region. However, as soon as the vector enters the target cell promoter conversion occurs, and the therapeutic and/or marker genes are expressed from a tissue specific promoter of choice introduced into the polylinker. Not only can virtually any tissue specific promoter be included in the system, providing for the selective targeting of a wide variety of different cell types, but additonally, following the conversion event, the structure and properties of the retroviral vector no longer resemble that of a virus. This, of course, has extremely important consequences from a safety point of view, since other retroviral vectors readily undergo genetic recombination with the retroviral packaging construct and/or endogenous retroviruses to produce potentially pathogenic viruses. Promoter conversion vectors do not resemble retroviruses because they no longer carry U3 retroviral promoters after conversion thus reducing the possibility of genetic recombination.

Rather than a tissue specific promoter a regulatable promoter can be inserted in the polylinker, allowing the conditional expression of genes carried by the retroviral vector.

Alternative to the promoter conversion vector the packaging cell according to the present invention can also be transfected with a reconstituting retroviral vector (see danish patent application no. 0005/98). This vector comprises one or more promoters inserted in antisense orientation within the 5' LTR region and one or more coding sequences inserted in antisense orientation within the 3' LTR region. Both, the promoter as well as the coding sequence, are inserted in such a way as to ensure that the promoter and the coding sequence become duplicated during the process of reverse transcription in a target cell and appear in the 3' as well as in the 5' LTR region of the resulting provirus in a fashion where the promoter is located upstream of the coding sequence allowing it to drive gene expression.

First of all, in the antisense orientation there is no gene following on the left (downstream) of the promoter. Additionally, the gene to be transferred and expressed in the target cell is non-functional because there is no promoter located to the right (upstream) of it.

After introduction of the vector into the packaging cell line, the retroviral vector is transcribed into RNA. This transcription is regulated by the normal unselective retroviral promoter contained in the U3 region of the 5' LTR (see above) and initiates at the boundary between the U3 and R region and terminates at the boundary between the R and U5 region of the 3' LTR. The RNA which represents the recombinant retroviral genome is packaged by the viral proteins produced by the packaging cell line according to the present invention to form MLV particles according to the present invention which bud from the cell. These particles are used to infect the target cell.

After infection of the target cell, the recombinant viral RNA will be reverse transcribed into DNA. During this process the promoter inserted in antisense orientation at the 5' end of the viral RNA will be duplicated and translocated to the 3' end of the generated DNA molecule, whilst the coding sequence(s) from the 3' end of the viral RNA will be duplicated and translocated to the 5' end of the DNA molecule.

As a result of this process the promoter and the gene are present twice within the target cells. Further, each copy of the gene is now linked to a copy of the promoter located upstream relative to the coding region allowing the gene to be expressed in the target cell. Consequently, this system avoids any leakiness of gene expression in the packaging cells, and allows expression of transferred genes in the target cell without the necessity for external stimuli.

In a preferred embodiment the promoter is inserted within the U5 region of the 5' LTR, preferably wholly or partly replacing the U5 region. Nevertheless, the promoter can also be inserted between the R and U5 region of the 5' LTR.

The gene coding sequences are especially inserted within the U3 region of the 3' LTR, preferably wholly or partly replacing the U3 region. Alternatively, they can be inserted between the R and U3 region of the 3' LTR.

The MLV particles according to the present invention are especially replication defective.

The MLV particles as well as the packaging cell are used for the treatment of diseases and for producing a pharmaceutical composition for *in vivo* and *in vitro* gene therapy in humans.

### Summary of the invention

The invention *inter alia* comprises the following alone or in combination:
a murine packaging cell for producing a retroviral particle, said cell comprising a DNA construct coding for proteins required for a mouse leukemia virus (MLV) derived vector to be packaged and a DNA construct coding for one or more human complement inhibitors, wherein one complement inhibitor is human CD59.
the cell as above wherein the MLV-derived vector comprises one or more heterologous nucleic acid sequences;
the cell as above wherein the heterologous sequence is a coding sequence;
the cell as above wherein the coding sequence encodes a substance applicable in gene therapy;
the cell as above wherein the coding sequence is selected from one or more elements of the group comprising marker genes, antiviral genes and/or antitumour genes;
the cell as above wherein the U3 region of the 3' long terminal repeat (3'LTR) of the retroviral vector is replaced by a polylinker sequence;
the cell as above wherein said polylinker sequence comprises at least one insertion of a heterologous nucleic acid fragment;
the cell as above wherein said heterologous nucleic acid fragment is selected from one or more elements of the group comprising regulatory elements and promoters;
the cell as above wherein said regulatory elements and promoters are target cell specific in their expression;
the cell as above wherein one or more promoters are inserted in antisense orientation within the 5'LTR region and one or more coding sequences are inserted in antisense orientation within the 3'LTR region of the retroviral vector, both, the promoter as well as the coding sequence, inserted in such a way as to ensure that the promoter and the coding sequence become duplicated during the process of reverse transcription in a target cell and appear in the 3' as well as in the 5' LTR region of the resulting provirus in a fashion where the promoter is located upstream of the coding sequence allowing it to drive gene expression;
the cell as above wherein said promoter is inserted within the U5 region of the 5'LTR;
the cell as above wherein said coding sequence is inserted within the U3 region of the 3'LTR;
a murine leukemia virus (MLV) particle produced by transfecting the packaging cell as above with a MLV-derived vector, said MLV particle comprising said complement inhibitor inserted in the viral envelope;
the MLV particle as above being replication-defective;
a method for producing a packaging cell as above wherein said cell is transfected with a DNA construct coding for one or more complement inhibitors, wherein one complement inhibitor is human CD59.
a method for producing a MLV particle as above wherein a packaging cell as above is transfected with a MLV-derived vector;
the packaging cell as above and/ or the MLV particle as above for use in the treatment of a disease;
use of the packaging cell as above and/or of the MLV particle as above for producing a pharmaceutical composition;
a pharmaceutical composition containing a therapeutically effective amount of the packaging cell as above and/or of the MLV particle as above;

### Example

The following example will further illustrate the present invention. It will be well understood by a person skilled in the art that the provided example in no way may be interpreted in a way that limits the applicability of the technology provided by the present invention to this example.

The regulatory proteins of the human complement system include CD59 (Protectin, HRF20, MIRL), CD55 (also known as decay accelerating factor, DAF) and CD46 (membrane cofactor protein, MCPI). Of all three factors it has been shown that CD59 gives the best protection against the complement system in the case of HIV-1. (Saifuddin et al., 1997). CD59 inhibits the final steps in production of an active MAC.

CD59 was overexpressed in virus producing PALSG/S cells which are derived from the murine packaging cell line PA317 (Miller and Buttimore, 1986) and which had been transfected with the retroviral vector pLXSNEGFP (Klein et al., 1997). Both, the CD59 expressing packaging cells and the recombinant virus produced by these cells show increased resistance to the lytic effects of human complement:

The coding region of CD59 was cloned as a cDNA into the expression vector pZeoSV (Invitrogen). The plasmid pZenNeoCD59 (Somerville et al., 1994) that carries the cDNA for the CD59 was digested with XhoI (Promega) and the resulting fragment of 1.2 kb cloned into the vector pZeoSV that had been digested with XhoI and then dephosphorylated using calf intestinal phosphatase (Promega). After a butanol precipitation (Thomas, 1994) the ligation was transformed into E. coli DH10B (Gibco) using electroporation (Dower et al., 1988). Transformed colonies were selected on Zeocin and plasmid DNA was isolated. The orientation of the insert was confirmed by sequence analysis using primers complementary to the CD59 gene as well as to the cloning vector. Two plasmids, namely pZeoSVCD59.1 and pZeoSVCD59.19, were obtained that carried the CD59 gene in the correct orientation.

Both plasmids were stably transfected into the cell line PALSG/S and, after selection on Zeocin, resistant colonies were pooled as two populations, PALSG/S-CD59.1 and PALSG/S-CD59.19. These populations, stably carrying the CD59 expression plasmid, were used for the following experiments:

To prove presence and activity of the CD59 protein these cells were treated with increasing amounts of human serum. Cells expressing CD59 should be more resistant to serum complement mediated lysis than parental murine cells.

1x10⁶ PALSG/S, PALSG/S-CD59.1, PALSG/S-CD59.19 and HeLa (human cervical carcinoma) cells were seeded in 10 cm dishes (Sarstedt) with 10 ml DMEM/10%FCS (Gibco). On the following day the medium was replaced by 2 ml of fresh DMEM/10%FCS containing 0, 100 or 400 µl of human serum. Serum was allowed to act upon the cells for 6 hours at 37°C, before it was diluted out by addition of 6 ml DMEM/10%FCS. On the next day the cells were counted.

Treatment with 100 µl human serum resulted in a lysis of more than 50% of parental PALSG/S cells, compared to the cell number obtained without human serum treatment (defined as 100%). In contrast, only 25% lysis was observed in the case of PALSG/S-CD59.1 and PALSG/-CD59.19 cells. A more pronounced effect was visible after treatment with 400 µl human serum. 90% of the parental PALSG/S cells were lysed, whereas the PALSG/S-CD59.1 cells only showed 50% lysis and PALSG/S-CD59.19 cells 30% lysis. As expected, human HeLa cells were not lysed, since these cells do not express α-gal. The increase in cell number observed with HeLa cells (about 120%) might be due to growth factors contained in the human serum.

These data indicate that transfection of the CD59 expression plasmid pZeoSV-CD59 into murine PALSG/S cells rendered them resistant to human complement mediated lysis.

Retroviruses incorporate host cell membrane as their envelope during the budding process. Thus, cellular proteins present in the membrane also become incorporated into virions. To investigate whether the CD59 protein expressed in PALSG/S transfected populations also becomes incorporated, recombinant virus particles from the PALSG/S-CD59.1 and PALSG/S-CD59.19 cells were evaluated for their resistance to complement mediated virolysis in comparison to virions budding from parental PALSG/S cells.

PALSG/S, PALSG/S-CD59.1 and PALSG/S-CD59.19 cells were grown to confluence in 10 cm dishes, before the medium was exchanged for 6 ml of fresh DMEM/10%FCS. On the next day the 24 h virus containing culture supernatant was filtered (0.45 µm pore size) and 100 µl mixed with 50, 100 or 250 µl of undiluted human serum. In parallel, assays were performed with 250 µl of heat inactivated human serum and without human serum. After incubation for one hour at 37°C, DMEM/10%FCS to a total volume of 2 ml, and Polybrene to 8 µg/ml was added. The virus containing cell culture supernatant was transfered to HeLa target cells seeded (6x10⁵) in 10 cm dishes on the previous day. After incubation for 5 hours at 37°C, 5% CO₂, 6 ml fresh DMEM/10%FCS was added. One day after the infection the cells were trypsinised and diluted. G418 selection was applied 24 hours later.

In 250 µl of human serum the unprotected virus showed a strong decrease (about 1000-fold) in titer resulting in only 5.0x10² cfu/ml, whereas the CD59 expressing packaging cells reach titers of 1.5x10³ cfu/ml and 5.3x10³ cfu/ml, respectively, equivalent to a 3-fold and a 11-fold better titer compared with parental PALSG/S cells. Similar, but less pronounced effects were observed with lower serum concentrations.

The above data show that CD59 is active on the cell surface after transfection of a CD59 expression construct and is incorporated into the virus envelope to protect virus from complement attack.

### References

Anderson, W.F. 1992, Human gene therapy. Science 256: 808-813.
Cooper, N.R., Jensen, F.C., Welsh, R.M., and Oldstone, M.B. 1976, J. Exp. Med. 144(4): 970-984.
Cosset, F.-L., Takeuchi, Y., Battini, J.-L., Weiss, R.A., and Collins, M.K.L. 1995, J. Virol. 69, 7430-7436.
Dower, W.J., Miller, J.F., and Ragsdale, C.W. 1988, Nucleic Acids Research 16(13): 6127-6145.
Galili, U., Shohet, S.B., Kobrins, E., Stults, C.L.M., and Macher, B.A. 1988, J. Biol. Chem. 263(33): 17755-17762.
Klein, D., Indraccolo, S., von Rombs, K., Amadori, A., Salmons, B., and Günzburg, W.H. 1997, Gene Therapy 4:1256-1260.
Kotani, H., P.B. Newton, S. Zhang, Y.L. Chiang, E. Otto, L. Weaver, R.M. Blaese, W.F. Anderson, and G.J. McGarrity 1994, Human Gene Therapy 5: 19-28.
Marschang, P., Sodroski, J., Würzner, R., and Dierich, M.P. 1995, Eur. J. Immunol. 25: 285-290.
Miller, A.D. and Buttimore, C. 1986, Molecular and Cellular Biology 6: 2895-2902.
Rosenberg, S.A., Anderson, W.F., Blaese, R.M. 1992, Hum. Gene Therapy 3: 75-90.
Rother, R.P. and Squinto, S.P. 1996, Cell 86:185-188.
Russell, D.W., Berger M.S., and Miller, D. 1995, Human Gene Therapy 6: 635-641.
Saifuddin, M., Hedayati, T., Atkinson, J.P., Holguin, M.H., Parker, C.J., and Spear, G.T. 1997: J. Gen. Virol. 78:1907-1911.
Somerville, C.A., Kyriazis, A.G., McKenzie, A., Allison, J., Pearse, M.J., and D'Apice, A.J. 1994, Transplantation 58(12): 1430-1435.
Spear, G.T., Lurain, N.S., Parker, C.J., Ghassemi, M., Payne, G.H., and Saifuddin, M. 1995, J. Immunol.: 4376-4381.
Takeuchi, Y., Cosset, F.-L., Lachmann, P,J., Okada, H., Weiss, R.A., and Collins, M.K.L. 1994, J. Virol. 68: 8001-8007.
Takeuchi, Y., Porter, C.D., Strahan, K.M., Preece, A.F., Gustafsson, K., Cosset, F.-L., Weiss, R.A., and Collins, K.L. 1996, Nature 379: 85-88.
Thomas, M.R. 1994, Biotechniques 16(6): 988-990.
Welsh, R.M., Cooper, N.R., Jensen, F.C., and Oldstone, M.B. 1975, Nature 257(5527): 612-614.

## Claims

1. A murine packaging cell for producing a retroviral particle, said cell comprising a DNA construct coding for proteins required for a mouse leukemia virus (MLV) derived vector to be packaged and a DNA construct coding for one or more human complement inhibitors, wherein one complement inhibitor is human CD59.

2. The cell according to claim 1, wherein the cell is PALSG/S.

3. The cell according to claim 1 or 2 wherein the MLV-derived vector comprises one or more heterologous nucleic acid sequences.

4. The cell according to claim 3 wherein the heterologous sequence is a coding sequence.

5. The cell according to claim 4 wherein the coding sequence encodes a substance applicable in gene therapy.

6. The cell according to claim 5 wherein the coding sequence is selected from one or more elements of the group comprising marker genes, antiviral genes and/or antitumour genes.

7. The cell according to any of claims 1 to 6 wherein the U3 region of the 3' long terminal repeat (3'LTR) of the MLV-derived vector is replaced by a polylinker sequence.

8. The cell according to claim 7 wherein said polylinker sequence comprises at least one insertion of a heterologous nucleic acid fragment.

9. The cell according to claim 8 wherein said heterologous nucleic acid fragment is selected from one or more elements of the group comprising regulatory elements and promoters.

10. The cell according to claim 9 wherein said regulatory elements and promoters are target cell specific in their expression.

11. The cell according to any of claims 1 to 6 wherein one or more promoters are inserted in antisense orientation within the 5'LTR region and one or more coding sequences are inserted in antisense orientation within the 3'LTR region of the retroviral vector, both, the promoter as well as the coding sequence, inserted in such a way as to ensure that the promoter and the coding sequence become duplicated during the process of reverse transcription in a target cell and appear in the 3' as well as in the 5' LTR region of the resulting provirus in a fashion where the promoter is located upstream of the coding sequence allowing it to drive gene expression.

12. The cell according to claim 11 wherein said promoter is inserted within the U5 region of the 5'LTR.

13. The cell according to claim 11 or 12 wherein said coding sequence is inserted within the U3 region of the 3'LTR.

14. A murine leukemia virus (MLV) particle produced by transfecting, the packaging cell according to any of claims 1 to 13 with a MLV-derived vector, said MLV particle comprising said complement inhibitor inserted in the viral envelope.

15. The MLV particle according to claim 14 being replication-defective.

16. A method for producing a packaging cell according to any of claims 1 to 13 wherein said cell is transfected with a DNA construct coding for one or more complement inhibitors, wherein one complement inhibitor is human CD59.

17. A method for producing a MLV particle according to any of claims 14 and 15 wherein a packaging cell according to any of claims 1 to 13 is transfected with a MLV-derived vector.

18. The packaging cell according to any of claims to 13 and/or the MLV particle according to any of claims 14 and 15 for use in the treatment of a disease.

19. Use of the packaging cell according to any of claims 1 to 13 and/or of the MLV particle according to any of claims 14 and 15 for producing a pharmaceutical composition.

20. A pharmaceutical composition containing a therapeutically effective amount of the packaging cell according to any of claims 1 to 13 and/or of the MLV particle according to any of claims 14 and 15.

## Patentansprüche

1. Maus-Verpackungszelle zur Herstellung eines retroviralen Partikels, wobei die Zelle ein DNA-Konstrukt umfasst, das für Proteine codiert, die zum Verpacken eines vom Maus-Leukämievirus (MLV) abgeleiteten Vektors erforderlich sind, sowie ein DNA-Konstrukt, das für einen oder mehrere menschliche Complement-Inhibitoren codiert, wobei ein Complement-Inhibitor menschliches CD59 ist.

2. Zelle gemäß Anspruch 1, wobei die Zelle PALSG/S ist.

3. Zelle gemäß Anspruch 1 oder 2, wobei der MLV-abgeleitete Vektor eine oder mehrere heterologe Nucleinsäuresequenzen umfasst.

4. Zelle gemäß Anspruch 3, wobei die heterologe Sequenz eine codierende Sequenz ist.

5. Zelle gemäß Anspruch 4, wobei die codierende Sequenz eine in der Gentherapie anwendbare Substanz codiert.

6. Zelle gemäß Anspruch 5, wobei die codierende Sequenz ausgewählt ist aus einem oder mehreren Elementen der Gruppe, umfassend Markergene, antivirale Gene und/oder Antitumorgene.

7. Zelle gemäß einem der Ansprüche 1 bis 6, wobei der U3-Bereich der 3' langen terminalen Sequenzwiederholung (3'LTR) des MLV-abgeleiteten Vektors durch eine Polylinkersequenz ersetzt ist.

8. Zelle gemäß Anspruch 7, wobei die Polylinkersequenz mindestens eine Insertion eines heterologen Nucleinsäurefragments umfasst.

9. Zelle gemäß Anspruch 8, wobei das heterologe Nucleinsäurefragment ausgewählt ist aus einem oder mehreren Elementen der Gruppe, umfassend Regulationselemente und Promotoren.

10. Zelle gemäß Anspruch 9, wobei die Regulationselemente und Promotoren in ihrer Expression für die Zielzelle spezifisch sind.

11. Zelle gemäß einem der Ansprüche 1 bis 6, wobei ein oder mehrere Promotoren in Antisinn-Ausrichtung im 5'LTR-Bereich eingefügt ist bzw. sind und eine oder mehrere codierende Sequenzen in Antisinn-Ausrichtung im 3'LTR-Bereich des retroviralen Vektors eingefügt ist bzw. sind, wobei sowohl der Promotor als auch die codierende Sequenz solcherart eingefügt sind, um sicherzustellen, dass der Promotor und die codierende Sequenz während des Verfahrens der Reverse Transcription in einer Zielzelle dupliziert werden und im 3'- sowie im 5'LTR-Bereich des resultierenden Provirus in einer Weise aufscheinen, bei der sich der Promotor stromaufwärts von der codierenden Sequenz befindet und es diesem dabei ermöglicht wird, die Genexpression voranzutreiben.

12. Zelle gemäß Anspruch 11, wobei der Promotor im U5-Bereich der 5'LTR eingefügt ist.

13. Zelle gemäß Anspruch 11 oder 12, wobei die codierende Sequenz im U3-Bereich der 3'LTR eingefügt ist.

14. Maus-Leukämievirus (MLV)-Partikel, hergestellt durch Transfizieren der Verpackungszelle gemäß einem der Ansprüche 1 bis 13 mit einem MLV-abgeleiteten Vektor, wobei das MLV-Partikel den Complement-Inhibitor in die Virushülle eingefügt umfasst.

15. MLV-Partikel gemäß Anspruch 14, wobei dieses replikationsdefekt ist.

16. Verfahren zur Herstellung einer Verpackungszelle gemäß einem der Ansprüche 1 bis 13, wobei die Zelle mit einem DNA-Konstrukt transfiziert wird, das für einen oder mehrere Complement-Inhibitoren codiert, wobei ein Complement-Inhibitor menschliches CD59 ist.

17. Verfahren zur Herstellung eines MLV-Partikels gemäß einem der Ansprüche 14 und 15, wobei eine Verpackungszelle gemäß einem der Ansprüche 1 bis 13 mit einem MLV-abgeleiteten Vektor transfiziert wird.

18. Verpackungszelle gemäß einem der Ansprüche 1 bis 13 und/oder MLV-Partikel gemäß einem der Ansprüche 14 und 15 zur Verwendung bei der Behandlung einer Erkrankung.

19. Verwendung der Verpackungszelle gemäß einem der Ansprüche 1 bis 13 und/oder des MLV-Partikels gemäß einem der Ansprüche 14 und 15 zur Herstellung einer pharmazeutischen Zusammensetzung.

20. Pharmazeutische Zusammensetzung, enthaltend eine therapeutisch wirksame Menge der Verpackungszelle gemäß einem der Ansprüche 1 bis 13 und/oder des MLV-Partikels gemäß einem der Ansprüche 14 und 15.

## Revendications

1. Cellule murine d'encapsidation pour produire une particule rétrovirale, ladite cellule comprenant une construction d'ADN codant pour des protéines nécessaires à un vecteur dérivé du virus de la leucémie murine (MLV) devant être encapsidé et une construction d'ADN codant pour un ou plusieurs inhibiteurs du complément humain, dans laquelle un inhibiteur du complément est le CD59 humain.

2. Cellule selon la revendication 1, la cellule étant une cellule PALSG/S.

3. Cellule selon la revendication 1 ou 2 dans laquelle le vecteur dérivé du MLV comprend une ou plusieurs séquences d'acides nucléiques hétérologues.

4. Cellule selon la revendication 3 dans laquelle la séquence hétérologue est une séquence codante.

5. Cellule selon la revendication 4 dans laquelle la séquence codante code pour une substance applicable en thérapie génique.

6. Cellule selon la revendication 5 dans laquelle la séquence codante est choisie parmi un ou plusieurs éléments du groupe comprenant des gènes marqueurs, des gènes antiviraux et/ou des gènes antitumoraux.

7. Cellule selon l'une quelconque des revendications 1 à 6 dans laquelle la région U3 de la longue répétition terminale en 3' (LTR 3') du vecteur dérivé du MLV est remplacée par une séquence de lieur multisite.

8. Cellule selon la revendication 7 dans laquelle ladite séquence de lieur multisite comprend au moins une insertion d'un fragment d'acide nucléique hétérologue.

9. Cellule selon la revendication 8 dans laquelle ledit fragment d'acide nucléique hétérologue est choisi parmi un ou plusieurs éléments du groupe comprenant des éléments régulateurs et des promoteurs.

10. Cellule selon la revendication 9 dans laquelle lesdits éléments régulateurs et promoteurs sont spécifiques d'une cellule cible dans leur expression.

11. Cellule selon l'une quelconque des revendications 1 à 6 dans laquelle un ou plusieurs promoteurs sont insérés dans l'orientation antisens à l'intérieur de la région LTR 5' et une ou plusieurs séquences codantes sont insérées dans l'orientation antisens à l'intérieur de la région LTR 3' du vecteur rétroviral, le promoteur ainsi que la séquence codante étant tous deux insérés de manière à garantir que le promoteur et la séquence codante soient dupliqués lors du processus de transcription inverse dans une cellule cible et apparaissent dans la région LTR 3' ainsi que la région LTR 5' du provirus résultant de façon à ce que le promoteur soit situé en amont de la séquence codante, lui permettant de réguler l'expression génique.

12. Cellule selon la revendication 11 dans laquelle ledit promoteur est inséré à l'intérieur de la région U5 du LTR 5'.

13. Cellule selon la revendication 11 ou 12 dans laquelle ladite séquence codante est insérée à l'intérieur de la région U3 du LTR 3'.

14. Particule de virus de la leucémie murine (MLV) produite en transfectant la cellule d'encapsidation selon l'une quelconque des revendications 1 à 13 avec un vecteur dérivé du MLV, ladite particule de MLV comprenant ledit inhibiteur du complément inséré dans l'enveloppe virale.

15. Particule de MLV selon la revendication 14 étant de réplication défectueuse.

16. Procédé de production d'une cellule d'encapsidation selon l'une quelconque des revendications 1 à 13 dans lequel ladite cellule est transfectée avec une construction d'ADN codant pour un ou plusieurs inhibiteurs du complément, dans lequel un inhibiteur du complément est le CD59 humain.

17. Procédé de production d'une particule de MLV selon l'une quelconque des revendications 14 et 15 dans lequel une cellule d'encapsidation selon l'une quelconque des revendications 1 à 13 est transfectée avec un vecteur dérivé du MLV.

18. Cellule d'encapsidation selon l'une quelconque des revendications 1 à 13 et/ou particule de MLV selon l'une quelconque des revendications 14 et 15 pour utilisation dans le traitement d'une maladie.

19. Utilisation de la cellule d'encapsidation selon l'une quelconque des revendications 1 à 13 et/ou de la particule de MLV selon l'une quelconque des revendications 14 et 15 pour produire une composition pharmaceutique.

20. Composition pharmaceutique contenant une quantité thérapeutiquement efficace de la cellule d'encapsidation selon l'une quelconque des revendications 1 à 13 et/ou de la particule de MLV selon l'une quelconque des revendications 14 et 15.
